(19) Europäisches Patentamt
European Patent Office
Office européen des brevets

(11) Numéro de publication: **0 093 664**
**B1**

# (12) FASCICULE DE BREVET EUROPEEN

(45) Date de publication du fascicule du brevet: **11.09.85**

(51) Int. Cl.⁴: **C 07 C 121/75**, C 07 C 120/00, A 01 N 53/00, A 23 K 1/16 // C07D307/93

(21) Numéro de dépôt: **83400853.4**

(22) Date de dépôt: **28.04.83**

(54) **Ester d'acide cyclopropane carboxylique et d'alcool(S)cyano(4-fluoro-3-phénoxyphényl) méthylique, sa préparation, son application à la lutte contre les parasites et les compositions le renfermant.**

(30) Priorité: **30.04.82 FR 8207485**

(43) Date de publication de la demande:
**09.11.83 Bulletin 83/45**

(45) Mention de la délivrance du brevet:
**11.09.85 Bulletin 85/37**

(84) Etats contractants désignés:
**BE CH DE GB IT LI NL**

(56) Documents cités:
**EP - A - 0 038 271**
**EP - A - 0 041 021**

**Le dossier contient des informations techniques présentées postérieurement au dépôt de la demande et ne figurant pas dans le présent fascicule.**

(73) Titulaire: **ROUSSEL-UCLAF, 35, boulevard des Invalides, F-75007 Paris (FR)**

(72) Inventeur: **Tessier, Jean, 30, rue Jean Moulin, F-94300 Vincennes (FR)**
Inventeur: **Demoute, Jean-Pierre, 249bis, rue de Rosny, F-93100 Montreuil-sous-Bois (FR)**
Inventeur: **Bonin, Werner, Im Schulzehnten 18, D-6233 Kelkheim (DE)**

(74) Mandataire: **Fritel, Hubert, ROUSSEL-UCLAF 111, route de Noisy Boîte Postale no. 9, F-93230 Romainville (FR)**

**EP 0 093 664 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

**Description**

On connaissait déjà d'après la demande de brevet européen 0 401 021 des composés de formule

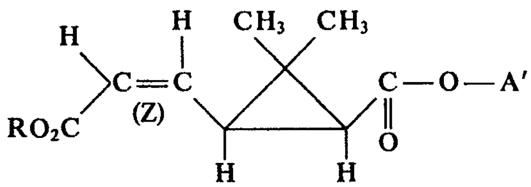

dans lequelle R représente un reste alcoyle linéaire, ramifié ou cyclique, saturé ou insaturé, A' représente notamment le reste d'un alcool utilisé en synthèse pyréthrinoïde et la double liaison a la géométrie Z.

La société demanderesse a maintenant découvert qu'un composé de cette famille, le 1R, cis 2,2-diméthyl 3—/Δ Z 3-oxo 3-terbutoxy propényl/cyclopropane carboxylate de (S)cyano (4-fluoro 3-phénoxyphényl)méthyle ou composé A, de formule:

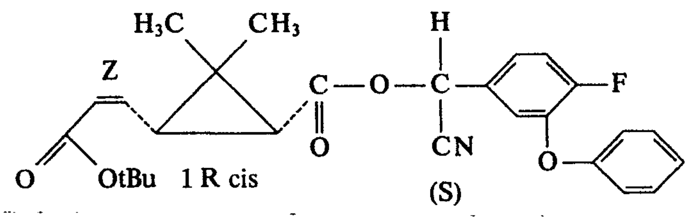

(A)

est doué de propriétés pesticides particulièrement intéressantes notamment dans les domaines acaricide et ixodicide comme le montrent les résultats des tests exposés ci-après. L'invention a donc pour objet le 1R, cis 2,2-diméthyl 3-/Δ Z 3-oxo 3-terbutoxy propényl/cyclopropane carboxylate de (S)cyano (4-fluoro 3-phénoxyphényl)méthyle.

L'invention a également pour objet un procédé de préparation du composé tel que défini précédemment, caractérisé en ce que l'on fait réagir, au sein d'un solvant organique, l'acide 1R, cis 2,2-diméthyl 3/Δ Z 3-oxo 3-terbutoxy propényl/cyclopropane carboxylique ou l'un de ses dérivés fonctionnels avec l'alcool (S)cyano (4-fluoro 3-phénoxyphényl)méthylique ou l'un de ses dérivés fonctionnels.

L'invention a notamment pour objet un procédé tel que défini précédemment, caractérisé en ce que l'on fait réagir l'acide (1R, cis) 2,2-diméthyl 3/Δ Z 3-oxo 3-terbutoxy propényl/cyclopropane carboxylique avec la 1-chloro N,N 2-triméthyl propényl amine puis soumet le produit résultant à l'action de l'alcool (S)cyano (4-fluoro-3-phénoxy phényl)méthylique, en présence de pyridine.

Dans la mise en oeuvre du procédé tel que défini précédemment, le solvant organique peut être choisi dans le groupe constitué par les solvants chlorés aliphatiques, les hydrocarbures aromatiques ou aliphatiques, cyclisés ou non et les éthers.

L'acide 1R, cis 2,2-diméthyl 3/Δ Z 3-oxo-3-terbutoxy propényl/cyclopropane carboxylique est décrit dans la demande de brevet européen 0 041 021. La préparation de l'alcool (S) cyano (4-fluoro-3-phénoxy phényl) méthylique est décrite dans la partie expérimentale.

Le composé A présente d'intéressantes propriétés qui permettent son utilisation dans la lutte contre les parasites; il peut s'agir, par exemple, de la lutte contre les parasites des végétaux, les parasites des locaux et les parasites des animaux à sang chaud. C'est ainsi que l'on peut utiliser le composé de l'invention pour lutter contre les insectes, les nématodes et les acariens parasites des végétaux et des animaux

L'invention a donc aussi pour objet l'application du composé A à la lutte contre les parasites des végétaux, les parasites des locaux et les parasites des animaux à sang chaud.

Le composé A peut donc être utilisé notamment pour lutter contre les insectes dans le domaine agricole, pour lutter par exemple contre les pucerons, les larves de lépidoptères et les coléoptères. Il est utilisé à des doses comprises entre 10 g et 300 g de matière active à l'hectare.

Le composé A peut également être utilisé pour lutter contre les insectes dans les locaux, pour lutter notamment contre les mouches, les moustiques et les blattes.

Le composé A est un produit tout à fait remarquable comme le montrent les résultats des tests ci-après. Il présente un excellent pouvoir létal et un très bon pouvoir de knock-down.

Le composé A est de plus photostable et n'est que peu toxique pour les mammifères.

L'ensemble de ces propriétés fait du composé A und produit qui correspond parfaitement aux exigences de l'industrie agrochimique moderne: il permet de protéger les récoltes tout en préservant l'environnement.

Le composé A peut aussi être utilisé pour lutter contre les acariens et les nématodes parasites des végétaux.

Le composé A peut encore être utilisé pour lutter contre les acariens parasites des animaux, pour lutter, par exemple, contre les tiques et notamment les tiques de l'espèce Boophilus ceux de l'espèce Hyalomnia, ceux de l'espèce Amblyomnia et ceux de l'espèce Rhipicephalus, ou pour lutter contre toutes sortes de gales et notamment la gale sarcoptique, la gale psoroptique et la gale chorioptique.

Le composé A peut aussi être utilisé dans la lutte contre les poux.

L'invention a donc également pour objet les compositions destinées à la lutte contre les parasites des végétaux, les parasites des locaux et les parasites des animaux à sang chaud, caractérisées en ce qu'elles renferment comme principe actif, le composé A. Ces compositions sont préparées selon les procédés usuels de l'industrie agrochimique.

L'invention a notamment pour objet les compositions insecticides renfermant comme principe actif le composé A.

Ces compositions insecticides peuvent être destinées à l'usage agricole, à l'usage dans les locaux pour lutter contre les parasites vecteurs de maladies, ou pour améliorer l'hygiène publique industrielle et domestique. Dans ces compositions insecticides, la matière active peut être additionnée éventuellemtent d'un ou plusieurs autres agents pesticides. Ces compositions peuvent se présenter sous forme de poudres, granulés, suspensions, émulsions, solutions, solutions pour aérosols, bandes combustibles, appâts ou autres préparations employés classiquement pour l'utilisation de ce genre de composés.

Outre le principe actif, ces compositions contiennent, en général, un véhicule et/ou un agent tensio-actif, non ionique, assurant, en outre, une dispersion uniforme des substances constitutives du mélange. Le véhicule utilisé peut être un liquide, tel que l'eau, l'alcool, les hydrocarbures ou autres solvants organiques, une huile minérale, animale ou végétale, une poudre telle que le talc, les argiles, les silicates, le kieselguhr ou un solide combustible.

Les compositions insecticides selon l'invention contiennent de préférence de 0,005% à 10% en poids de matière active.

Selon un mode opératoire avantageux, pour un usage dans les locaux, les compositions selon l'invention sont utilisées sous forme de compositions fumigantes.

Les compositions selon l'invention peuvent alors être avantageusement constituées, pour partie non active, d'un serpentin combustible ou encore d'un substrat fibreux incombustible présenté sous forme de plaquette. Dans ce dernier cas, le fumigant obtenu après incorporation de la matière active est placé sur un appareil à résistance chaufante.

Dans le cas où l'on utilise un serpentin insecticide, le support inerte peut être, par exemple, composé de marc de pyrèthre, poudre de Tabu (ou poudre de feuilles Machilus Thumbergii), poudre de tige de pyrèthre, poudre de feuille de cèdre, poudre de bois (telle que la sciure de pin) amidon et poudre de coque de noix de coco.

La dose de matière active peut alors être, par exemple de 0,03% à 1% en poids.

Dans le cas où l'on utilise un support fibreux incombustible, la dose de matière active peut alors être, par exemple, de 0,03% à 95% en poids.

Les compositions selon l'invention pour un usage dans les locaux, peuvent aussi être obtenues en préparant une huile pulvérisable à base de principe actif, cette huile imbibant la mèche d'une lampe et étant alors soumise à la combustion.

La concentration du principe actif incorporé à l'huile est de préférence de 0,03% à 95% en poids.

L'invention a aussi notamment pour objet les compositions acaricides destinées à la lutte contre les parasites des végétaux, renfermant comme principe actif, le composé A.

L'invention a également pour objet les compositions nématicides renfermant comme principe actif, le composé A.

Les compositons insecticides selon l'invention, comme les compositions acaricides et nématicides peuvent être additionnés éventuellement d'un ou plusieurs autres agents pesticides.

Les compositions acaricides et nématicides peuvent se présenter notamment sous forme de poudres, granulés, suspensions, émulsions, solutions.

Pour l'usage acaricide, on utilise de préférence des poudres mouillables pour pulvérisation foliaire, contenant de 1 à 80% de principe actif ou des liquides pour pulvérisation foliaire contenant de 1 à 500 g/l de principe actif. On peut également employer des poudres pour poudrage foliaire contenant de 0,05 à 3% de matière active.

Pour l'usage nématicide, on utilise de préférence des liquides pour traitement des sols contenant de 300 à 500 g/l de principe actif.

Le composé acaricide et nématicide selon l'invention est utilisé de préférence à des doses comprises entre 1 à 100 g de matière active à l'hectare.

L'invention a également pour objet les compositions acaricides, destinées à la lutte contre les parasites des animaux à sang chaud tels que poux, les gales et notamment les compositions ixodicides destinées à la lutte contre les ixodes tels que les tiques. Elles sont administrées le plus souvent par la voie externe (vaporisation, shampooing, bain, badigeonnage et plus praticulièrement badigeonnage de l'épine dorsale) selon la méthode dite »pour-on«. Elles peuvent également être administrées par voie digestive.

Pour exalter l'activité biologique du produit de l'invention, on peut dans ses différentes applications,

l'additionner à des synergistes classiques utilisés en pareil cas tel que le 1(2,5,8-trioxadodécyl)2-propyl 4,5-méthylènedioxy benzène (ou butoxyde de pipéronyle) ou la N-(2-éthyl heptyl) bicyclo/2,2-1/5-hepten-2,3-dicarboximide ou le pipéronylbis-2-(2'-n-butoxy éthoxy)éthylacétal (ou tropital).

Lorsqu'il s'agit de lutter contre les acariens parasites des animaux domestiques, on peut incorporer le produit de l'invention dans des compositions alimentaires en association avec un mélange nutritif adapté à l'alimentation animale. Le mélange nutritiel peut varier selon l'espèce animale; il peut renfermer des céréales, des sucres et des grains, des tourteaux de soja, des farines de poissons, des acides aminés de synthèse, des sels minéraux, des vitamines et des antioxydants.

L'invention a donc aussi pour objet les compositions destinées à l'alimentation animale renfermant comme principe actif le composé A. Ces compositions sont préparées selon les procédés usuels de l'industrie des produits destinés à la nutrition animale.

L'invention a également pour objet les associations douées d'activité insecticide, acaricide ou nématicide, caractérisées en ce qu'elles contiennent comme matière active, d'une part le composé A, et d'autre part, un au moins des esters pyréthrinoïdes choisis dans le groupe constitué par les esters d'alléthrolones, d'alcool 3,4,5,6-tétrahydrophtalimido méthylique, d'alcool 5-benzyl 3-furyl méthylique, d'alcool 3-phénoxy benzylique et d'alcools $\alpha$-cyano 3-phénoxy benzyliques des acides chrysanthémiques, par les esters d'alcool 5-benzyl 3-furyl méthylique des acides 2,2-diméthyl 3-(2-oxo 3-tétrahydrothiophénylidène méthyl) cyclopropane-1-carboxyliques, par les esters d'alcool 3-phénoxy benzylique et d'alcools $\alpha$-cyano 3-phénoxy benzyliques des acides 2,2-diméthyl 3-(2,2-dichlorovinyl) cyclopropane-1-carboxyliques, par les esters d'alcools $\alpha$-cyano 3-phénoxy benzyliques d'acides 2,2-diméthyl 3-(2,2-dibromovinyl) cyclopropane-1-carboxyliques, par les esters d'alcool 3-phénoxy benzylique des acides 2-parachlorophényl 2-isopropyl acétiques, par les esters d'alléthrolones, d'alcool 3,4,5,6-tétrahydrophtalimido méthylique, d'alcool 5-benzyl 3-furyl méthylique, d'alcool 3-phénoxy benzylique et d'alcools $\alpha$-cyano 3-phénoxy benzyliques des acides 2,2-diméthyl 3-(1,2-2,2-tétrahaloéthyl) cyclopropane-1-carboxyliques, dans lesquels »halo« représente un atome de fluor, de chlore ou de brome, étant entendu que les copules acides et alcools des esters pyréthrinoïdes ci-dessus peuvent exister sous toutes leurs formes stéréoisomères possibles.

Les exemples suivants illustrent l'invention.

## Exemple

### 1R, cis 2,2-diméthyl 3/($\Delta$Z)3-oxo 3-terbutoxy propényl/cyclopropane carboxylate de (S)cyano (4-fluoro-3-phénoxy phényl)méthyle

Dans 10 cm$^3$ de dichlorométhane on introduit 1,25 cm$^3$ de 1-chloro NN 2-triméthyl propényl amine, agite à 20° C, introduit progressivement une solution de 1,45 g d'acide 1R, cis 2,2-diméthyl 3/($\Delta$Z)3-oxo 3-terbutoxy propényl/cyclopropane carboxylique dans 5 cm$^3$ de dichlorométhane, agite pendant 30 minutes, introduit lentement une solution contenant 1,45 g d'alcool (S)-cyano (4-fluoro 3-phénoxyphényl)méthylique, 1,2 cm$^3$ de pyridine 10 cm$^3$ de dichlorométhane, agite à 20°C pendant 18 heures, ajoute de l'eau, une solution aqueuse diluée d'acide chlorhydrique, sépare par décantation la phase organique, la lave à l'eau, la concentre à sec par distillation sous pression réduite, purifie le résidu par chromatographie sur silice en éluant par un mélange d'hexane et d'acétate déthyle (9/1), cristallise dans l'hexane et obtient 2,04 g de 1R, cis 2,2-diméthyl 3/($\Delta$Z) 3-oxo 3-terbutoxy propényl/cyclopropane carboxylate de (S)cyano (4-fluoro 3-phénoxy phényl)méthyle.

F = 114°C $(\alpha)_D = +67° \pm 2,5°$.(c = 0,5% chloroforme).

Spectre de RMN (deutérochloroforme):

- pics à 1,23—1,27 ppm attribués aux hydrogènes des méthyles géminés,
- pic à 1,52 ppm attribué aux hydrogènes du terbutyle,
- pics à 1,87—2,0 ppm attribués à l'hydrogène en position 1 du cyclopropyle,
- pics à 3,2—3,5 ppm attribués à l'hydrogène en position 3 du cyclopropyle,
- pics à 5,8—6,0 ppm attribués à l'hydrogène éthylénique en $\alpha$ du carboxyle,
- pics à 6,3—6,6 ppm attribués à l'autre hydrogène éthylénique,
- pic à 6,35 ppm attribué à l'hydrogène porté par le même carbone que le groupement cyano,
- pics de 6,98 à 7,6 ppm attribués aux hydrogènes des noyaux aromatiques.

L'alcool (S)-cyano (4-fluoro 3-phénoxyphényl)méthylique utilisé au départ de l'exemple prédédent peut être préparé comme suit:

Stade A

(1R, 2R, 5S) 6,6-diméthyl 3-oxa-2-/(R)-cyano(3-phénoxy 4-fluorophényl)méthoxy/bicyclo (3.1.0) hexane et (1R, 2R, 5S)6,6-diméthyl 3-oxa-2-/(S)-cyano (3-phénoxy 4-fluorophényl)méthoxy/bicyclo (3.1.0)hexane

On mélange 16 g d'alcool (RS)-cyano (4-fluoro 3-phénoxyphényl)méthylique, 100 $cm^3$ de dichlorométhane, 9,4 g de (1R, 2R, 5S)6,6-diméthyl 3-oxa bicyclo (3.1.0) hexan-2-ol, 0,1 g d'acide paratoluène sulfonique, porte le mélange réactionnel au reflux, maintient le reflux pendant une heure et trente minutes, verse dans une solution aqueuse diluée de bicarbonate de potassium, sépare et concentre la phase organique à sec par distillation sous pression réduite et obtient 25,06 g de (1R, 2R, 5S)6,6-diméthyl 3-oxa 2-(RS)-cyano (4-fluoro 3-phénoxyphényl)méthoxy/bicyclo (3.1.0)hexane.

On chromatographie le produit obtenu sur silice en éluant par le mélange hexane-éther (8/2) et obtient 8,85 g du (1R, 2R, 5S)6,6-diméthyl 3-oxa 2-/(R)-cyano (4-fluoro 3-phénoxyphényl) méthoxy/bicyclo (3.1.0)hexane.

F < 50° C, $(\alpha)_D$ = +102° (c = 1% benzène).

Les caractéristiques de ce produit sont les suivantes:

Dichroïsme circulaire (dioxane)

— max. à 279 nm $\Delta\varepsilon = -0,27$.

Spectre de RMN (deutérochloroforme):

— pic à 1,07 ppm attribué aux hydrogènes des méthyles géminés,
— pics à 1,33—1,78 ppm attribués aux hydrogènes du cyclopropyle,
— pics à 3,7—4,1 ppm attribués aux hydrogènes de $-CH_2O$,
— pics à 5,2—5,5 ppm attribués aux hydrogènes des

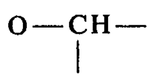

— pics à 6,9—7,6 ppm attribués aux hydrogènes des noyaux aromatiques.

En continuant la chromatographie, on obtient 9,05 g du (1R, 2R, 5S)6,6-diméthyl 3-oxa-2-/(S)-cyano (4-fluoro 3-phénoxyphényl)méthoxy/bicyclo (3.1.0) hexane.

F = 65° C $(\alpha)_D$ = +50° (c = 0,4% benzène).

Les caractéristiques de ce produit sont les suivantes:

Dichroïsme circulaire (dioxane):
— Inflexion à 275 nm $\Delta\varepsilon = +0,13$; max. à 281 nm $\Delta\varepsilon = +0,15$.

Spectre de RMN (deutérochloroforme):

— pic à 1,0 ppm attribué aux hydrogènes des méthyles géminés,
— pics à 1,55—1,57 ppm attribués aux hydrogènes du cyclopropyle,
— pics à 3,8—3,9 ppm et 4,1—4,3 ppm attribués aux hydrogènes de $-CH_2-O-$
— pics à 4,9—5,3 ppm attribués aux hydrogènes des $O-CH <$
— pics à 6,9—7,6 ppm attribués aux hydrogènes des noyaux aromatiques.

Stade B

Alcool (S)-cyano (4-fluoro 3-phénoxyphényl)méthylique

On mélange 9 g de (1R, 2R, 5S)6,6-diméthyl 3-oxa-2-/(S)-cyano-(3-phénoxy 4-fluoro phényl)méthoxy/bicyclo (3.1.0)hexane, 100 $cm^3$ de méthanol, 90 mg d'acide paratoluène sulfonique, agite à 20°C pendant une heure et trente minutes, verse le mélange réactionnel dans l'eau, extrait au chloroforme, concentre la phase organique à sec par distillation sous pression réduite, chromatographie le résidu sur silice en éluant dans un mélange d'hexane et d'acétate d'éthyle (7/3) à 1% d'acide acétique et obtient 4,5 g de produit recherché.

$(\alpha)_D$ = −30° (c = 0,5% pyridine).

## Exemples de compositions selon l'invention

### Exemple A

Préparation d'un concentré soluble

On effectue un mélange homogène de:

| | |
|---|---|
| — composé selon l'invention | 0,25 g |
| — butoxyde de pipéronyle | 1 g |
| — Tween 80 | 0,25 g |
| — Topanol A * 2,4-diméthyl 6-terbutyl phénol | 0,1 g |
| — Eau | 98,4 g |

— 2,4-diméthyl 6-terbutyl phénol.

### Exemple B

Préparation d'un concentré émulsifiable

On mélange intimement:

| | |
|---|---|
| — Composé selon l'invention | 0,015 g |
| — butoxyde de pipéronyle | 0,5 g |
| — topanol A | 0,1 g |
| — xylène | 95,885 g |
| — tween 80 | 3,5 g |

### Exemple C

Préparation d'un concentré émulsifiable

On effectue un mélange homogène de:

| | |
|---|---|
| — composé selon l'invention | 1,5 g |
| — tween 80 | 20 g |
| — topanol A | 0,1 g |
| — xylène | 78,4 g |

### Exemple D

Préparation d'une composition fumigène

| | |
|---|---|
| — composé selon l'invention | 0,25 g |
| — poudre de tabu | 25 g |
| — poudre de feuille de cèdre | 40 g |
| — poudre de bois de pin | 33,75 g |
| — vert brillant | 0,5 g |
| — p-nitrophénol | 0,5 |

## Etude de l'activité insecticide du composé de l'invention

### a) Etude de l'effet létal sur mouche domestique

Les insectes tests sont des mouches domestiques femelles âgées de 4 à 5 jours. On opère par application topique de 1 il de solution acétonique sur le thorax dorsal des insectes à l'aide du micro manipulateur d'Arnold. On utilise 50 individus par dose testée. On effectue le contrôle de mortalité vingt-quatre heures après traitement.

Le résultat obtenu exprimé en DL 50 (ou dose en nanogrammes par individu nécessaire pour tuer 50% des insectes) est le suivant:

DL 50 en ng par individu = 1,8.

b) Etude de l'effet d'abattage sur mouche domestique

Les insectes tests sont des mouches domestiques femelles âgées de 4 jours. On opère par pulvérisation directe à la concentration de 0,25 g/l en chambre de Kearns et March en utilisant comme solvant un mélange d'acétone (5%) et d'Isopar L (solvant pétrolier) (quantité de solution délivrée dans l'expérience : 2 ml en une seconde). On utilise 50 insectes par traitement. On effectue les contrôles toutes les minutes jusqu'à 10 minutes, puis à 15 minutes et l'on détermine le KT 50 par les méthodes habituelles.

Le résultat expérimental obtenu est le suivant:

KT 50 en minutes = 3,8.

c) Etude de l'effet létal sur blatte

Les tests sont effectués par contact sur film de verre, après dépôt à la pipette de solutions acétonique de différentes concentrations sur fond de boîte de Pétri en verre dont les bords ont été préalablement talqués afin d'éviter la fuite des insectes. On détermine la concentration létale 50 (CL 50).

Le résultat expérimental obtenu est le suivant:

CL 50 en $mg/m^2$ = 0,087.

d) Etude de l'effet létal sur Acanthoscelides obtectus

Les essais sont effectués par application topique d'une solution acétonique à l'aide du micro manipulateur d'Arnold. Après traitement, les individus adultes sont placés sur un milieu naturel. On effectue le contrôle des mortalités 48 heures après traitement.

Le résultat expérimental obtenu est le suivant:

DL 50 en mg/individu = 8,3.

e) Etude de l'effet létal sur Aphis carcivora

On utilise des jeunes adultes agés de 2 à 3 jours et l'on emploie 10 individus par concentration utilisée. On pratique dans ce cas un test de contact-ingestion: après traitement au pistolet de Fisher avec une solution toxique, une feuille de fève est déposée dans une boite de Pétri en matière plastique sur une rondelle de papier humidifiée. Le traitement est effectué à l'aide de 2 ml d'un mélange d'eau, d'acétone et de produit à tester (1 ml par face de feuille). L'infestation de la feuille par les insectes est effectuée après séchage de la feuille. Ce test de contact-ingestion dure pendant une heure puis on place les insectes sur des feuilles non traitées et l'on contrôle la mortalité au bout de 24 heures.

Le résultat expérimental obtenu est le suivant:

DL 50 en mg par/l de solution = 5,2.

Ainsi dans les tests précédents, le composé de l'invention apparait comme doué d'une très bonne activité insecticide et d'un excellent effet d'abattage.

Etude de l'activité acaricide du composé de l'invention

On utilise des plants de haricot comportant 2 feuilles cotylédonaires.

Les plants sont traités au pistolet Fisher: 4 ml de solution toxique par plan d'un mélange à volume égal d'eau et d'acétone. On laisse sécher pendant 12 heures puis on procède à l'infestation. L'infestation est effectuée à l'aide de 25 femelles de Tetranychus urticae par feuille soit 50 femelles par plant et par dose testée. Chaque plan est mis sous bonette aérée et disposée sous plafond lumineux en lumière constante. Les contrôles de mortalité sont effectués 80 heures après. La dose utilisée dans chaque test est de 5 g de produit par hl.

On détermine la CL 50 en mg par hectolitre.

Le résultat expérimental obtenu est le suivant:

CL 50 en mg/hl = 536.

Dans ce test le composé de l'invention montre donc une excellente activité acaricide.

Etude de l'activité ixodicide du composé de l'invention

a) Etude de l'activité sur larve de Boophilus microplus

La substance à tester est dissoute dans un mélange de phénylsulfonate CA 70 (50 g), d'Emulsogen

EL 360 (70 g) et d'Isophoron (780 g) de façon à obtenir un concentré émulsifiant à 10%. On dilue ce concentré avec de l'eau pour obtenir des solutions de concentration souhaitée de 10 ppm, 1 ppm et 0,1 ppm.

Au moyen d'une tour de pulvérisation, on pulvérise les différentes solutions ci-dessus sur des larves de tiques de boeufs des tropiques, de type Boophilus microplus (souches Mexico sensible et DDT résistante) et on détermine après 24 heures, par comptage des larves mortes et vivantes, le pourcentage de mortalité. Sur ce test les pourcentages de mortalité sont 100%.

b) Etude de l'activité sur tiques adultes
Rhipicephalus appendiculatus et Amblyomma hebraeum

On utilise des concentrations 100, 10 et 1 pm. Les solutions sont pulvérisées comme ci-dessus. On utilise 10 tiques par concentration. Les résutats sont exprimés en pourcentage de mortalité. Sur ce test les pourcentages de mortalité sont de 100%.

c) Etude de l'activité du produit sur l'inhibition de la reproduction de tiques
Boophilus microplus (Souche Mexico sensible)

On plonge pendant 5 minutes des femelles de Boophilus microplus prêtes à pondre, dans les solutions préparées cidessus (voir point a) puis on les porte dans une enceinte chauffée pour la ponte.

On détermine $\alpha$) le pourcentage de tiques n'ayant pas pondu 2 semaines après traitement, $\beta$) la quantité d'oeufs pondus 2 semaines après traitement en fonction d'un témoin, $\gamma$) le pourcentage de larves ayant éclos, après 3 semaines.

On calcule, en fonction des chiffres obtenus, le pourcentage d'inhibition de la reproduction; 100% signifie que l'inhibition est totale et 0% que la reproduction est identique à celle obtenue avec les témoins.

A la dose de 0,19 ppm, le pourcentage d'inhibition trouvé a été de 100%.

Conclusion

Le composé de l'invention présente une très bonne activité ixodicide.

**Revendications**

1. Le 1R, cis 2,2-diméthyl 3/$\Delta$Z 3-oxo 3-terbutoxy propényl/cyclopropane carboxylate de (S)-cyano (4-fluoro 3-phénoxy phényl)méthyle.

2. Procédé de préparation du composé tel que défini à la revendication 1, caractérisé en ce que l'on fait réagir au sein d'un solvant organique, l'acide 1R, cis 2,2-diméthyl 3/$\Delta$Z 3-oxo 3-terbutoxy propényl/cyclopropane carboxylique ou l'un de ses dérivés fonctionnels avec l'alcool (S)-cyano (4-fluoro 3-phénoxy phényl)méthylique ou l'un de ses dérivés fonctionnels.

3. Procédé tel que défini à la revendication 2, caractérisé en ce que l'on fait réagir l'acide (1R, cis) 2,2-diméthyl 3/$\Delta$Z 3-oxo 3-terbutoxy propényl/cyclopropane carboxylique avec la 1-chloro N,N 2-triméthyl propényl amine, puis soumet le produit résultant à l'action de l'alcool (S)-cyano (4-fluoro 3-phénoxyphényl(méthylique, en présence de pyridine.

4. Procédé selon l'une des revendications 2 ou 3, caractérisé en ce que le solvant organique est choisi dans le groupe constitué par les solvants chlorés aliphatiques, les hydrocarbures aromatiques ou aliphatiques, cyclisés ou non et les éthers.

5. Application du composé tel que défini à la revendication 1 à la lutte contre les parasites des végétaux, les parasites des locaux et les parasites des animaux à sang chaud.

6. Les compositions destinées à la lutte contre les parasites des végétaux, les parasites des locaux et les parasites des animaux à sang chaud, caractérisées en ce qu'elles renferment comme principe actif le produit défini à la revendication 1.

7. Les compositions insecticides renfermant comme principe actif le produit défini à la revendication 1.

8. Les compositions acaricides renfermant comme principe actif le composé défini à la revendication 1.

9. Les compositions acaricides destinées à la lutte contre les acariens parasites des animaux et notamment contre les ixodes renfermant comme principe actif le composé défini à la revendication 1.

10. Les compositions nématicides renfermant comme principe actif le composé défini à la revendication 1.

11. Les compositions destinées à l'alimentation animale renfermant comme principe actif le produit défini à la revendication 1.

12. Associations douées d'activité insecticide, acaricide ou nématicide, caractérisées en ce qu'elles contiennent comme matière active, d'une part le composé défini à la revendication 1 et d'autre part, un au moins des esters pyréthrinoïdes choises dans le groupe constitué par les esters d'alléthrolones, d'alcool 3,4,5,6-tétrahydrophtalimido méthylique, d'alcool 5-benzyl 3-furyl méthylique, d'alcool 3-phénoxy benzylique et d'alcools α-cyano 3-phénoxy benzyliques des acides chrysanthémiques, par les esters d'alcool 5-benzyl 3-furyl méthylique des acides 2,2-diméthyl 3-(2-oxo 3-tétrahydrothiophénylidène méthyl)cyclopropane-1-carboxyliques, par les esters d'alcool 3-phénoxy benzylique et d'alcools α-cyano 3-phénoxy benzyliques des acides 2,2-diméthyl 3-(2,2-dichlorovinyl)cyclopropane-1-carboxyliques, par les esters d'alcools α-cyano 3-phénoxy benzyliques d'acides 2,2-diméthyl 3-(2,2-dibromovinyl) cyclopropane-1-carboxyliques, par les esters d'alcool 3-phénoxy benzylique des acides 2-parachlorphényl 2-isopropyl acétiques par les esters d'alléthrolones, d'alcool 3,4,5,6-tétrahydrophtalimido méthylique, d'alcool 5-benzyl 3-furyl méthylique, d'alcool 3-phénoxy benzylique et d'alcools α-cyano 3-phénoxy benzyliques des acides 2,2-diméthyl 3-(1,2,2,2-tétrahaloéthyl) cyclopropane-1-carboxyliques, dans lesquels »halo« représente un atome de fluor, de chlore ou de brome, étant entendu que les copules acides et alcools des esters pyréthrinoïdes cidessus peuvent exister sous toutes leurs formes stéréoisomères possibles.

13. Les compositions telles que définies à l'une quelconque des revendications 6 à 12, caractérisées en ce qu'elles renferment en outre un synergiste.

**Patentansprüche**

1. (S)-Cyano-(4-fluor-3-phenoxyphenyl)-methyl-1R,cis-2,2-dimethyl-3[ΔZ-3-oxo-3-tert.-butoxy-propenyl]-cyclopropan-carboxylat.

2. Verfahren zur Herstellung der Verbindung gemäß Anspruch 1, dadurch gekennzeichnet, daß man in dem Medium eines organischen Lösungsmittels die 1R,cis-2,2-Dimethyl-3-[ΔZ-3-oxo-3-tert.-butoxy-propenyl]-cyclopropan-carbonsäure oder eines ihrer funktionellen Derivate mit dem (S)-Cyano-(4-fluor-3-phenoxyphenyl)-methylalkohol oder einem seiner funktionellen Derivate umsetzt.

3. Verfahren gemäß Anspruch 2, dadurch gekennzeichnet, daß man die (1R,cis)-2,2-Dimethyl-3-[ΔZ-3-oxo-3-tert.-butoxy-propenyl]-cyclopropan-carbonsäure mit 1-Chlor-N,N-2-trimethylpropenylamin umsetzt und dann das entstandene Produkt der Einwirkung von (S)-Cyano-(4-fluor-3-phenoxyphenyl)-methylalkohol in Gegenwart von Pyridin unterzieht.

4. Verfahren nach einem der Ansprüche 2 oder 3, dadurch gekennzeichnet, daß das organische Lösungsmittel unter aliphatischen chlorierten Lösungsmitteln, cyclisierten oder nichtcyclisierten aromatischen oder aliphatischen Kohlenwasserstoffen und Äthern ausgewählt wird.

5. Verwendung der Verbindung gemäß Anspruch 1 bei der Bekämpfung von Parasiten der Pflanzen, Parasiten von Räumlichkeiten und Parasiten warmblütiger Tiere.

6. Zusammensetzungen für die Bekämpfung von Parasiten der Pflanzen, Parasiten von Räumlichkeiten und Parasiten warmblütiger Tiere, dadurch gekennzeichnet, daß sie als Wirkstoff das Produkt gemäß Anspruch 1 enthalten.

7. Insektizide Zusammensetzungen enthaltend als Wirkstoff die Verbindung gemäß Anspruch 1.

8. Akarizide Zusammensetzungen enthaltend als Wirkstoff die Verbindung gemäß Anspruch 1.

9. Akarizide Zusammensetzungen für die Bekämpfung von parasitären Milben der Tiere, und insbesondere von Zecken, enthaltend als Wirkstoff die Verbindung gemäß Anspruch 1.

10. Nematizide Zusammensetzungen enthaltend als Wirkstoff die Verbindung gemäß Anspruch 1.

11. Zusammensetzungen für die tierische Ernährung enthaltend als Wirkstoff das Produkt gemäß Anspruch 1.

12. Mit insektizider, akarizider oder nematizider Aktivität ausgestattete Assoziationen, dadurch gekennzeichnet, daß sie als Wirkstoff einesteils die Verbindung gemäß Anspruch 1 und anderenteils zumindest einen der Pyrethrinoidester enthalten, ausgewählt unter den Estern der Allethrolone, des 3,4,5,6-Tetrahydrophthalimido-methylalkohols, des 5-Benzyl-3-furyl-methylalkohols, des 3-Phenoxy-benzylalkohols und der α-Cyano-3-phenoxy-benzylalkohole der Chrysanthemumsäuren, unter den Estern des 5-Benzyl-3-furyl-methylalkohols der 2,2-Dimethyl-3-(2-oxo-3-tetrahydrothiophenylidenmethyl)-cyclopropan-1-carbonsäuren, unter den Estern des 3-Phenoxy-benzylalkohols und der α-Cyano-3-phenoxy-benzylalkohole der 2,2-Dimethyl-3-(2,2-dichlorvinyl)-cyclopropan-1-carbonsäuren, unter den Estern der α-Cyano-3-phenoxy-benzylalkohole der 2,2-Dimethyl-3-(2,2-Dibromvinyl)-cyclopropan-1-carbonsäuren, unter den Estern des 3-Phenoxy-benzylalkohols der 2-p-Chlorphenyl-2-isopropyl-essigsäuren, unter den Estern der Allethrolone, des 3,4,5,6-Tetrahydrophthalimido-methylalkohols, des 5-Benzyl-3-furyl-methylalkohols, des 3-Phenoxy-benzylalkohols und der α-Cyano-3-phenoxy-benzylalkohole der 2,2-Dimethyl-3-(1,2,2,2-Tetrahaloäthyl)-cyclopropan-1-carbonsäuren, worin »Halo« ein Fluor-, Chlor- oder Bromatom bedeutet, wobei die sauren und alkoholischen Verknüpfungskomponenten der vorstehenden Pyrethrinoidester in sämtlichen ihrer möglichen stereoisomeren Formen vorliegen können.

13. Zusammensetzungen gemäß einem der Ansprüche 6 bis 12, dadurch gekennzeichnet, daß sie außerdem einen Synergisten enthalten.

## Claims

1. 1R, cis 2,2-dimethyl 3[ΔZ-3-oxo-3-tert-butoxypropenyl]cyclopropane carboxylate of (S)-cyano(4-fluoro 3-phenoxyphenyl)methyl.

2. Preparation process for the compound as defined in Claim 1, characterized in that 1R, cis 2,2-dimethyl 3[ΔZ-3-oxo-3-tert-butoxy-propenyl]cyclopropane carboxylic acid or one of its functional derivatives is made to react in an organic solvent with (S)-cyano (4-fluoro-3-phenoxyphenyl)methyl alcohol or one of its functional derivatives.

3. Process as defined in Claim 2, characterized in that (1R, cis) 2,2-dimethyl-3[ΔZ-3-oxo-3-tert-butoxy propenyl]-cyclopropane carboxylic acid is made to react with 1-chloro N,N-2-trimethylpropenylamine, then the resulting product is submitted to the action of (S)-cyano-(4-fluoro-3-phenoxyphenyl)methyl alcohol in the presence of pyridine.

4. Process according to any one of the Claims 2 or 3, characterized in that the organic solvent is chosen from the group composed of the aliphatic chlorated solvents, the aromatic or aliphatic hydrocarbons, cyclized or not, and the ethers.

5. Use of the compound as defined in Claim 1 for combatting parasites of vegetation, parasites of premises and parasites of warm-blooded animals.

6. Compositions intended for combatting parasites of vegetation, parasites of premises and parasites of warmblooded animals, characterized in that they contain as active principle the product defined in Claim 1.

7. Insecticide compositions containing as active principle the product defined in Claim 1.

8. Acaricide compositions containing as active principle the compound defined in Claim 1.

9. Acaricide compositions intended for combatting parasitic acarids of animals and in particular ticks, containing as active principle the compound defined in Claim 1.

10. Nematocide compositions containing as active principle the compound defined in Claim 1.

11. Compositions intended for animal alimentation containing as active principle the product defined in Claim 1.

12. Combinations endowed with insecticide, acaricide or nematocide activity, characterized in that they contain as active material, on the one hand the compound defined in Claim 1 and on the other hand, one at least of the pyrethrinoid esters chosen from the group constituted by the esters of allethrolones, of 3,4,5,6-tetrahydrophthalimidomethyl alcohol, of 5-benzyl-3-furyl methyl alcohol, of 3-phenoxybenzyl alcohol and of α-cyano-3-phenoxybenzyl alcohols with chrysanthemic acids, by the esters of 5-benzyl-3-furyl methyl alcohol with 2,2-dimethyl-3-(2-oxo-3-tetrahydrothiophenylidenemethyl) cyclopropane-1-carboxylic acids, by the esters of 3-phenoxybenzyl alcohol and of α-cyano-3-phenoxybenzyl alcohols with 2,2-dimethyl-3-(2,2-dichlorovinyl)cyclopropane-1-carboxylic acids, by the esters of α-cyano-3-phenoxybenzyl alcohols with 2,2-dimethyl-3-(2,2-dibromovinyl)cyclopropane-1-carboxylic acids, by the esters of 3-phenoxybenzyl alcohol with 2-parachlorophenyl-2-isopropyl acetic acids, by the esters of allethrolones, of 3,4,5,6-tetrahydrophthalimidomethyl alcohol, of 5-benzyl-3-furyl methyl alcohol, of 3-phenoxybenzyl alcohol and of α-cyano-3phenoxybenzyl alcohols with 2,2-dimethyl-3-(1,2,2,2-tetrahaloethyl)cyclopropane-1-carboxylic acids, in which »halo« represents a fluorine, chlorine or bromine atom, it being understood that the acid and alcohol copulas of the above pyrethrinoid esters can exist in all their possible stereo-isomeric forms.

13. Compositions as defined in any one of the Claims 6 to 12, characterized in that they also contain a synergist.